# EUROPEAN PATENT SPECIFICATION

(11) **EP 0 664 107 B1**
(45) Date of publication and mention of the grant of the patent: **02.12.1998**
(21) Application number: 95100498.5
(22) Date of filing: 16.01.1995
(51) Int. Cl.: A61F 2/06

(54) **Vascular prosthesis and device for its application**
Gefässprothesen sowie Vorrichtung zum Anbringen
Prothéses vasculaires et dispositif d'application

(30) Priority: 19.01.1994 IT MI940066
(43) Date of publication of application: 26.07.1995
(73) Proprietor: NAZARI, Stefano, I-22100 Como (IT)
(72) Inventor: NAZARI, Stefano, I-22100 Como (IT)
(74) Representative: Modiano, Guido, Dr.-Ing.

(56) References cited:
- EP-A- 0 544 485
- DE-A- 2 805 749

## Description

The present invention consists of a vascular prosthesis for the substitution or internal lining of blood vessels of medium and large diameter and of a device for its application.

Aneurisms of various origin and traumatic lesions are the most frequent conditions requiring thoracic and abdominal aortic prosthetic substitution. Current technique includes the surgical exposure of the aortic segment to be replaced; both ends of the aneurysm are isolated and clamped to stop the blood flow; the vessel is opened and a tubular conduit of biocompatible material is interposed and manually sutured at both ends.

Most complications of descending thoracic aorta prosthetic substitution (paraplegia, liver and renal injury) are due to ischemic lesions of distal organs (spinal cord, liver and kidney) during the blood flow interruption phase and the incidence is proportional to its duration. Other complications are related to the manoeuvres taken to compensate the haemodynamic modifications induced by clamping (extracorporeal circulation, heparinization, etc.)

A further source of complications is the suture line which can bleed at the operative table or cause pseudoaneurysm in the late postoperative period; these problems are enhanced by the alterations of the aortic wall due to the primary disease.

DE-A-2805749 discloses a prosthetic graft for repairing a damaged aorta segment, comprising in collapsed formation an elongated tube with a plurality of folds extending longitudinally and mounted on upper and lower convoluted expansion rings. Slip rings passing through the tube material and around the expansion rings permit the latter to "unwind" while still holding the tube in place. The tube is collapsed around a carrier line, as a catheter tube fitted with a head to facilitate the movement of the tube with the graft there-around through the blood vessel. Disposed inside the catheter is a wire which leads to a positioning hook, and coupled with the catheter by a plurality of slip rings is an extension lead wire, coupled with the expansion rings. The catheter is guided to the location of the aneurysm where the positioning hook is moved outwardly by pushing on the lead wire. When, the wire is pushed against the convoluted expansion rings thereby causing the convolutions to move apart and form a singular ring of larger diameter. The slip rings permit the expansion rings to move relative to the folded expansion tube. Expansion wire may be withdrawn by pulling away from the location of the aneurysm to disconnect the wire. Also, the positioning hook is retracted to its initial position beneath the head thereby allowing the catheter with wires attached to be withdrawn.

The main aim of this invention is to solve the above mentioned problems using a vascular prosthesis that can be applied very quickly greatly reducing the duration of the interruption of the blood flow in the segment to be substituted.

Within the above aim, an object of the invention is that of realizing a prosthesis that can be applied also without blood flow interruption.

Another aim of the invention is that of realizing a prosthesis that can be applied without requiring necessarily the surgical exposition of the blood vessel.

Another aim of the invention is to realize a prosthesis that can be applied to the vessel without surgical suture.

A further aim is that of realizing a device that would allow the positioning and the application of the vascular prosthesis, as invented, without interruption of the blood flow in the substituted vascular segment.

This aim and these and other objects which will become apparent hereinafter are achieved according to the invention by a vascular prosthesis for the substitution or internal lining of blood vessels of medium and large diameter comprising a tubular body of biocompatible material including, in proximity of at least one of its longitudinal ends, with at least one body at annular development disposed substantially coaxial to said tubular body and radially expandible for the engagement of the tract of said tubular body, containing said body at annular development, against the internal walls of a blood vessel proximal to the segment to be substituted or internally lined, with the prosthesis, said body at annular development being constituted of at least one wire elastically flexible slidingly mounted on a support associated to a lateral portion of said tubular body, said wire describing, internally to said tract of the tubular body, a double loop with a first tract coming out from said support along a direction inclined in respect to a plane which is perpendicular to the axis of said tubular body, with a second tract at annular development linked to said support in a zone spaced from the zone of coming out of said first tract, said second tract of the double loops developing in a plane substantially perpendicular to the axis of said tubular body, and with a third tract coming back into said support in a zone proximal to the zone of coming out of the said first tract form the support, said wire being sliding relatively to said support in order to vary the amplitude of said double loop, tools being provided for contrast of the sliding of said wire relatively to said support in order to maintain the expansion or contraction given to said double loop by the controlled sliding of said wire relatively to said support, at the ceasing of the force generating the sliding.

For the application of the vascular prosthesis according to the invention, when it is desired to operate without blood flow interruption, a device is preferably used, comprising a flexible tubular support insertable into the lumen of a blood vessel distal to the tract to be substituted or internally lined with said prosthesis, retaining tools for said support, provided at the distal end of said tubular support predisposed to be inserted into the blood vessel, tools for deactivation of said retaining tools for said support provided in connection with said retaining tools, command tools provided at the proximal end of said tubular support predisposed to lie externally to said vessel, and connecting tools, connected to said command tools, for connection of said command tools to said at least one wire, said command tools including a first handle and a second handle connected to the proximal end of said tubular support and said connection tools including a first cable extending internally to said tubular support for connection to said at least one wire and a second cable extending internally to said tubular support to prevent the sliding off of said first cable from said tubular support, said first handle being connected to said first cable and said second handle being connected to said second cable, said first and second handle being jointly movable relatively to said tubular support for the sliding of said at least one wire relatively to said support for a variation of the amplitude of said at least one double loop for the radial expansion or contraction of said body at annular development, or individually movable relatively to said tubular support to disconnect said at least one wire form the command tools, thereby leaving the prosthesis internally to the vessel.

Further features and advantages of the invention will be apparent from the following description of a preferred, though not exclusive embodiment of the vascular prosthesis according to the invention, as well as of a device for its application, illustrated in the accompanying illustrative, not limitative drawings wherein:

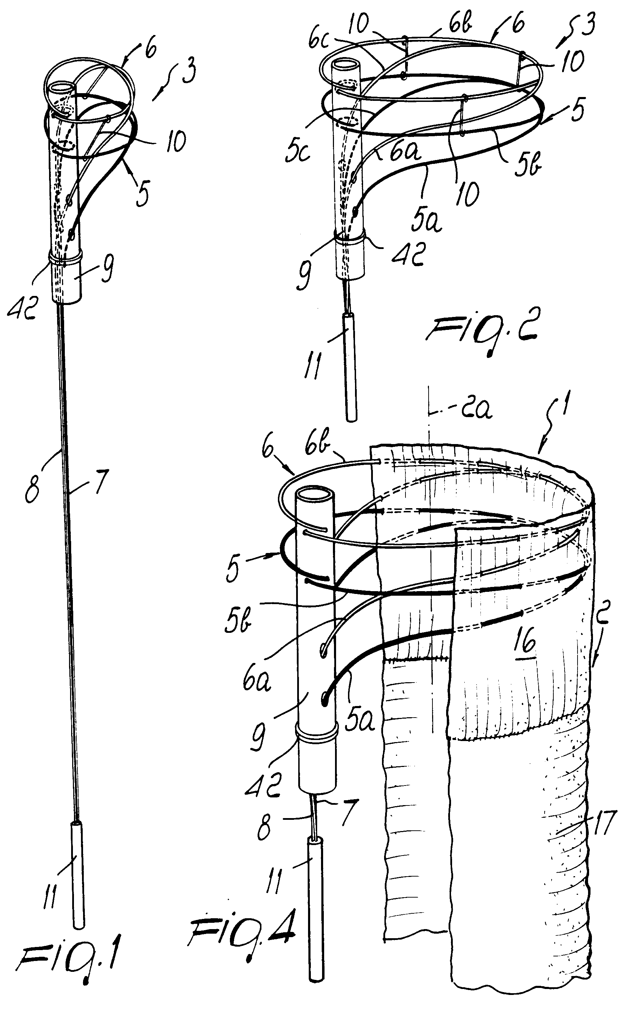

figure 1 illustrates the body at annular development of the prosthesis according to the invention in perspective view in position radially contracted;

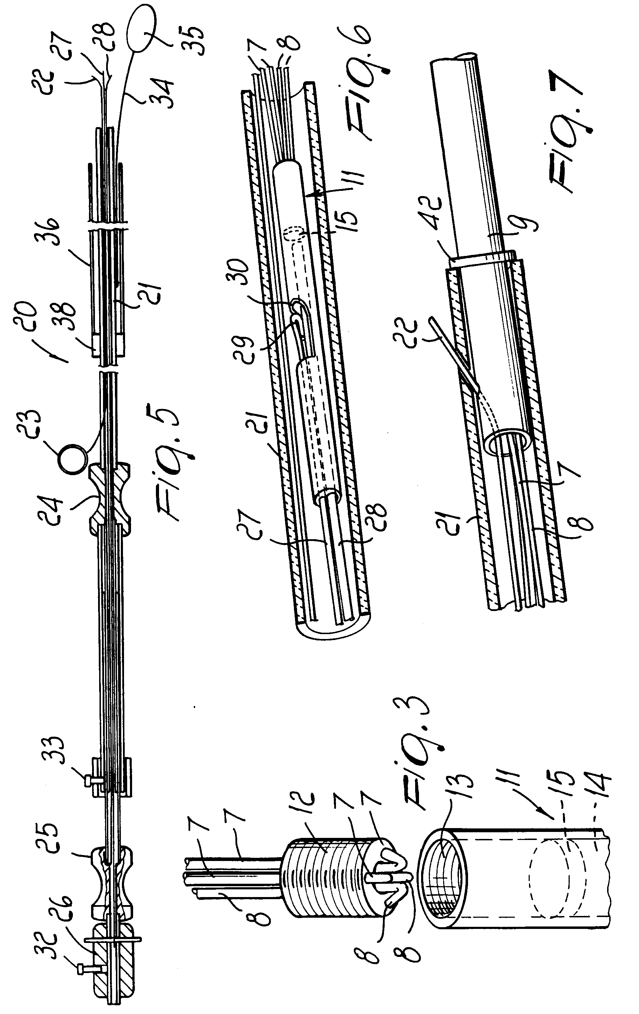

figure 2 illustrates the body at annular development of the prosthesis according to the invention in perspective view in position radially expanded;
figure 3 shows a particular enlarged and exploded of figures 1 and 2;

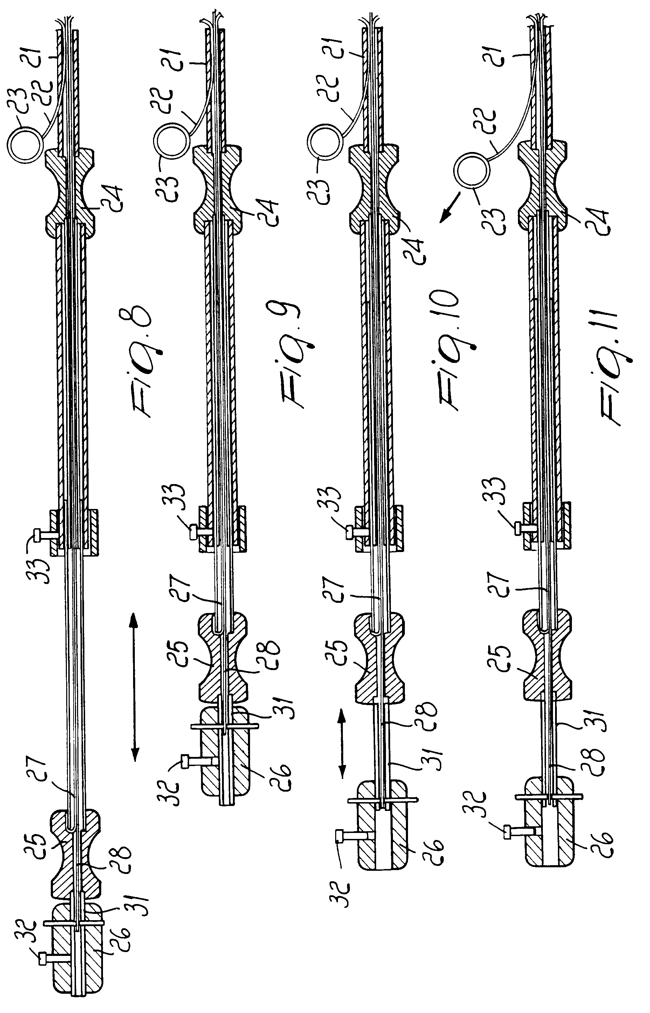

figure 4 illustrates a detail enlarged and partially in section of the prosthesis according to the invention;
figure 5 is a schematic axial section of the device for the application of the prosthesis according to the invention;
figure 6 illustrates a detail enlarged, in perspective and partially sectioned view, of the device for the application of the prosthesis according to the invention;
figure 7 illustrates a detail enlarged, in perspective and partially sectioned view, of the device;
figures between 8 and 11 illustrate the device in proximity to its proximal end in different functioning conditions;
figure 12 schematicly illustrates the insertion of the prosthesis according to the invention into a blood vessel;
figure 13 is an enlarged section of figure 12 carried out along the axis XIII-XIII;
figure 14 illustrates the radial expansion phase of the body at annular development of the prosthesis inside the blood vessel;
figure 15 illustrates a detail enlarged of figure 14;

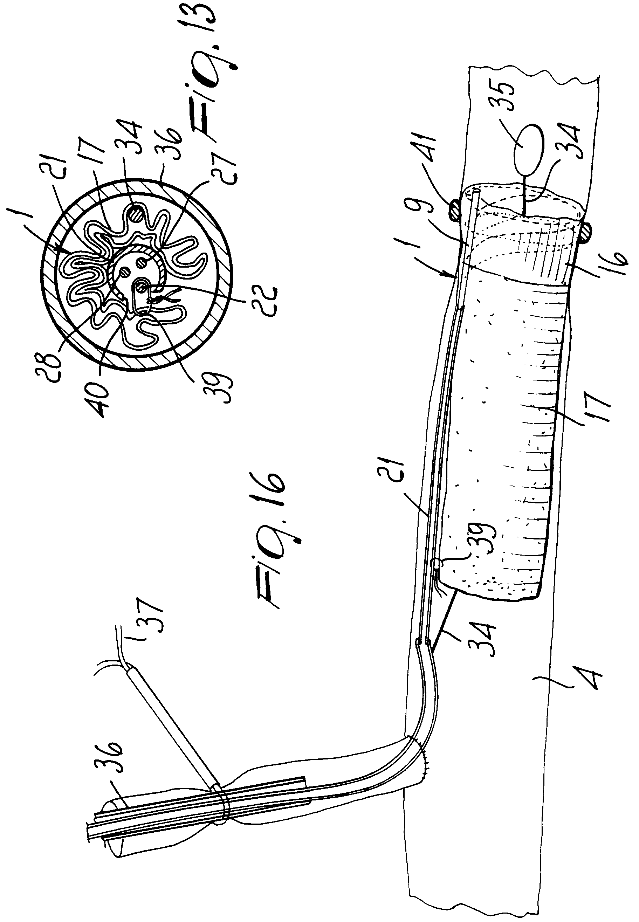

figure 16 illustrates the device and the prosthesis at the end of its application;
figure 17 illustrates, in perspective view partially sectioned, a variant of execution of the prosthesis according to the invention.

With reference to the cited figures, the vascular prosthesis according to the invention, indicated generally by the reference numeral 1, comprises a tubular body 2 realized in synthetic biocompatible material, for example with materials commercially known as dacron or teflon, that is associated, in correspondence of at least one of its longitudinal ends, with at least one body at annular development 3 which is disposed substantially coaxial to the tubular body 2 and which is radially expandible in order to obtain the engagement of the tract of the tubular body 2, which contains the body at annular development 3, against the inner walls of a blood vessel 4 proximal to the tract of the vessel to be substituted or internally lined with the prosthesis.

According to the invention, the body at annular development 3 consists of at least one double loop 5, 6 of a wire 7, 8 which is elastically flexible and which is mounted in a sliding way on a support 9 associated to a lateral portion of the tubular body 2 parallel to the axis 2a of tubular body 2. The wire 7, 8 is sliding relatively to the support 9 in order to vary the amplitude of double loop 5, 6 or to radially increase or decrease the occupancy of the tract of tubular body 2 that contains the double loop 5, 6, and tools are provided to contrast the sliding of wire 7, 8 relatively to the support 9, in such a way that it is maintained the expansion or the contraction, given to the double loop 5, 6 by means of the controlled sliding of wire 7, 8 relatively to the support 9, at the ceasing of the force generating the sliding.

Preferably two wires 7, 8, in harmonic stainless steel are provided, which describe a double loop each, respectively 5 and 6 and each of these double loops presents: a first tract 5a, 6a which comes out from the support 9 along a direction which is inclined in respect to an ideal plane transversal to the axis 2a of the tubular body 2, a second tract 5b, 6b, at annular development, which is bound to the support 9 in a zone that is spaced out of the zone from which comes out the first tract 5a, 6a, and which develops in a plane which is substantially perpendicular to the axis 2a of the tubular body 2, and a third tract 5c, 6c that re-enter into the support 9 in a zone that is proximal to the zone from which comes out the first tract 5a, 6a.

The support 9 is preferably constituted by a body substantially cylindric, preferably hollow, and wires 7, 8 come out laterally from the support 9 to make the double loops 5, 6.

Advantageously, the double loops 5, 6 are spaced between them in a direction which is parallel to the axis 2a of the tubular body 2, or in direction parallel to the axis 9a of the support 9, spacing the holes through whom the tracts of wires 7, 8 come out laterally from the support 9.

It should be noted that the connection of the second tract 5b, 6b of the double loops 5, 6 to the support 9 is obtained by means of multiple passages of wire 7, 8 which make the double loops 5, 6 through the support 9 in a position which is properly spaced from the position of the holes through whom wires 7, 8 come out from support 9 in such a way that the second tract 5b, 6b of the loops is maintained in a plane which is substantially perpendicular to the axis 2a of the tubular body 2 without significant variation during the use.

The tools for contrast of the sliding of the wires 7, 8 relatively to the support 9, in order to maintain the given radial expansion or contraction of the double loops 5, 6, are constituted by the friction resulting from the passage through the support 9 of the wires 7, 8. This friction, which is depending from the dimension and configuration of the holes defined in the support 9 and through whom the wires 7, 8 pass, as well as from the material constituting the support 9 in relation with the material by which are realized the wires 7, 8, can provide in any way a sufficient resistance to undesired accidental contraction or expansion of the double loops 5, 6.

Advantageously tools are provided for the positioning of one of the double loops 5 in respect to the other double loop 6. These tools for positioning comprise segments of wire 10, also made of harmonic stainless steel, that connect each other the second tract 5b of the double loop 5 with the second tract 6b of the double loop 6 in such a way that they contrast efficiently the flexions of the second tract 5b, 6b of the loops and they maintain them in planes substantially parallel to each other and substantially perpendicular to the axis 2a of the tubular body 2.

The ends of the wires 7 and 8 that constitute the loops 5 and 6 come out from an axial end of the support 9 and are associated, jointly, to a little cylinder block of manoeuvre 11 that is mobile at command relatively to the support 9 in order to cause the sliding of wires 7, 8 to obtain a variation of the amplitude of the double loops 5 and 6.

More in detail, the four ends of the wires 7 and 8 are connected to the little cylinder block of manoeuvre 11 by inserting them previously in a sleeve 12, screw-threaded outside, that gives room just sufficient to contain the four ends of the wires 7 and 8, and folding, after having passed the sleeve 12, the ends of wires 7 and 8, in such a way that is prevented their sliding off the sleeve 12. The sleeve 12 is coupled with a screw-threaded side 13 realized at one end of the little cylinder block of manoeuvre 11, preferably conformed in a substantially cylindrical shape. At the other end of the little cylinder block of manoeuvre 11 is realized a cavity 14, delimited by a diaphragm 15 that divides it from the screw-threaded side 13.

The double loops 5, 6 of the wire 7, 8 and at least the portion of the support 9 which is interested by the double loops 5, 6 are connected, for example by repeated crossing passages, to a first portion 16 of the tubular body 2 that consists preferably of a segment of prosthesis of fabric disposed at annular configuration in such a way that the folds usually present in these fabrics result to be parallel to the axis 2a and then the radial contraction and the expansion of this portion 16 is facilitated. This first portion 16 is associated, for example by a suture, to the axial end of a second portion substantially cylindric 17 which is substantially coaxial to the first portion 16 in its annular disposition.

The little cylinder block of manoeuvre 11 and the portion of the wires 7, 8 that extend from little cylinder block of manoeuvre 11 to the support 9 are disposed externally to the tubular body 2.

The device for the application of the prosthesis according to the invention, indicated generally by the reference numeral 20, comprises a flexible tubular support, or sheath 21, that is insertable into the lumen of a blood vessel 4 distal to the tract to be substituted or internally lined with the prosthesis 1. This tubular support 21 is fitted, at its distal end, that is devised to be inserted into the blood vessel 4, with tools for retaining the support 9 and, at the proximal end that is devised to lie externally to the vessel 4, with control tools that are connected, through connecting tools that can slide inside the tubular support 21, to wires 7 and 8 to cause their sliding relatively to the support 9.

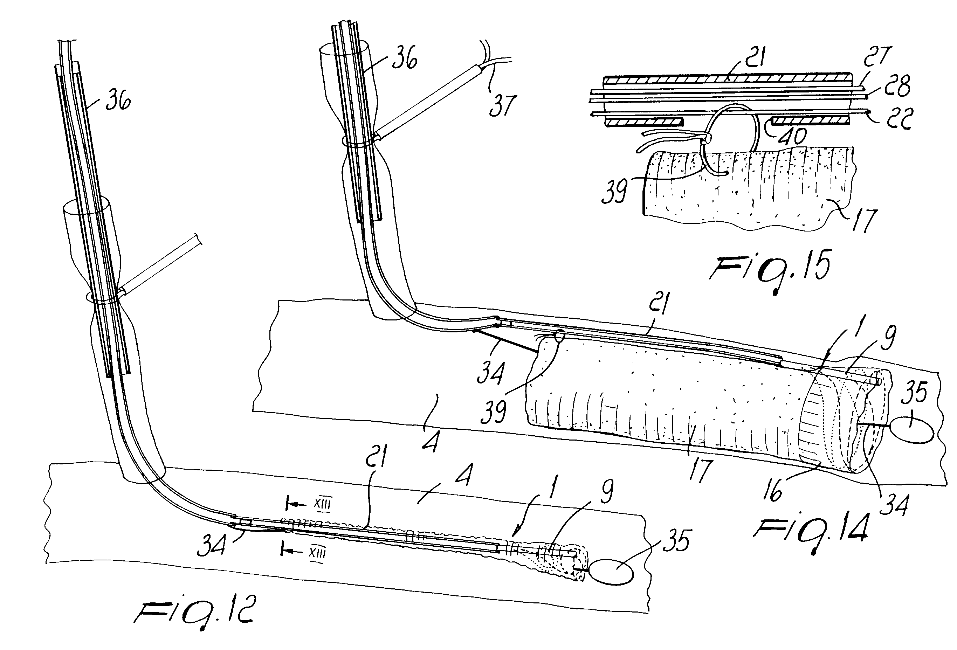

The tools for retaining the support 9 include, advantageously, a little retaining cable 22 which is slideable inside the tubular support 21 and that crosses predisposed holes realized at the distal end of the tubular support 21 and at that part of the end of the support 9 that is advantageously inserted inside the distal end of the tubular support 21; in this way the little retaining cable 22, by passing through both the support 9 and the tubular support 21, connects each other these two organs, contrasting the axial sliding of one in respect to the other, as shown in detail in figure 7. The axial sliding of the support 9 in direction of the tubular support 21 can be further contrasted by a beat ring 42 in a zone of the support 9 proximal to its end to be inserted into the tubular support 21.

The other end of the little retaining cable 22 comes out from the tubular support 21, in a zone of it that is close to its proximal end and therefore it lies, during the operation, externally to the blood vessel 4, and is fitted with a ring 23 that allows to cause the sliding of the little retaining cable 22 with respect to the tubular body 21 in the direction that cause the release of that little cable from the support 9 and from the tubular support 21 thus releasing the support 9 from the tubular support 21.

Advantageously in proximity to the ring 23, on the tubular support 21, a zone for handling 24 is provided in order to enhance the action of traction of the little retaining cable 22.

The controlling tools, in order to cause the sliding of the wires 7 and 8 relatively to the support 9, include a first handle 25 and a second handle 26 that are sited in correspondence of the proximal end of the tubular support 21 and that are connected to the little cylinder block of manoeuvre 11 by a first cable 27 and a second cable 28 that can slide internally to the tubular support 21 following the axial movement of the handles 25 and 26 in respect to tubular support 21.

More in detail, the first cable 27 presents, in correspondence with its distal end, i.e. the opposite end with respect to the handle 25, a folding 29 which brings its whole occupancy to be equal to two times the diameter in correspondence of this folding 29. The first cable 27 is inserted into the end of the little cylinder block of manoeuvre 11 opposite to the end from which is inserted the sleeve 12 and the folding 29 is sited in correspondence of a deficit 30 prepared in an intermediate zone of the lateral surface of the little cylinder block of manoeuvre 11. The cavity 14 presents, from the inserting end of the first cable 27 till the deficit 30, a lumen equal to nearly twice the diameter of the first cable 27, in such a way that the first cable 27 can be inserted and removed in the little cylinder block of manoeuvre 11 in spite of the presence of the folding 29. In the little cylinder block of manoeuvre 11 from the same end of the insertion of the first cable 27, is inserted, after the insertion of the first cable 27, the second cable 28 that, with its presence, prevents the sliding off of the first cable 27.

The two handles 25 and 26 can jointly slide axially relatively to the tubular support 21 so as to operate the axial sliding of the little cylinder block of manoeuvre 11 in respect to support 9 retained at distal end of the tubular support 21 to cause a variation of the amplitude of the double loops 5 and 6, otherwise they can slide one in respect to the other so as to allow the release of the second cable 28 from the little cylinder block of manoeuvre 11 and then the release of the first cable 27 from the little cylinder block of manoeuvre 11 so as to release the little cylinder block of manoeuvre 11 from the tubular support 21 when it is desired to leave the prosthesis in the blood vessel.

The first handle 25 presents, advantageously, a prolongation 31 which extends itself inside the handle 26 and that can be blocked by a screw 32, so as to solidarize the handle 25 and 26 between them, or so as to release one handle from the other according to the necessity.

Moreover, on the proximal end of the tubular support 21 is predisposed a screw 33 that allows to block the sliding of the first handle 25 relatively to the tubular support 21.

Advantageously, tools are provided for the guidance of the prosthesis into the blood vessel. Such tools include a cable guide 34 that is connected with its end to the tubular support 21 and that crosses axially the tubular body 2 of the prosthesis 1. The end of this cable guide 34, opposite to the end connected to the tubular support 21, is provided with an ogival body 35.

The tubular support 21 is mounted in an axially sliding way and blood-tightly, inside a tube 36 that is partially insertable inside the vessel or in a branch that can be closed around the tube 36, for example by a ligature 37, so as to realize the blood tightness during the insertion of the prosthesis 1 into the vessel 4.

The tightness between the tubular support 21 and the tube 36 can be obtained by positioning inside the tube 36 a disk gasket 38 which is crossed by the tubular support 21.

In order to avoid blood losses toward the external, tight disks 48 are provided also between the internal surface of the tubular support 21 and the cables 22, 27 and 28.

Advantageously tools for anchoring the tubular body 2 of the prosthesis to the lateral surface of the tubular support 21 during the insertion are also provided. Such anchoring tools include a button-hole 39 that can be obtained by a segment of thread for suture, that is connected to the tubular body 2 of the prosthesis in proximity to its end opposite to the tract 16 and that is insertable into a predisposed fissure 40 realized in the lateral surface of the tubular support 21 in correspondence with the passage of the retaining cable 22, in such a way that the retaining cable 22 passes also through the button-hole 39 causing in such a way the lateral anchoring of the prosthesis that can be leaved in site by the partial sliding of the retaining cable 22 from the tubular support 21, as illustrated in detail in fig 13 and 15.

The application of the prosthesis according to the invention with the device described above is carried out as follows.

First of all the ogival body 35 is passed axially through the prosthesis with its first portion 16 properly dilated.

Then the prosthesis 1 is disposed in the conditions of minimal occupancy around its axis 2a by manually moving as much as possible the little cylinder block of manoeuvre 11 from the support 9, so that the loops 5 and 6 are maximally contracted. The little cylinder block of manoeuvre 11 is then linked to the end of the first cable 27 by disposing the folding 29 in correspondence of the deficit 30 and by inserting into the cavity 14 the second cable 28 in such a way that this cable 28 keeps blocked the first cable 27 into the little cylinder block of manoeuvre 11. At this point the little cylinder block of manoeuvre 11 is inserted into the distal end of the tubular support 21 till a tract of the end of the support 9 results also inserted in the distal end of the tubular support 21. The support 9 is then linked to the tubular support 21 by the insertion, through the support 9 and through the flexible support 21, of the end of the retaining cable 22. During the manoeuvre of insertion of the retaining cable 22, the button-hole 39 sited in proximity of the proximal end of the prosthesis, is inserted into the fissure 40 and crossed by the same retaining cable 22. In this way the prosthesis is still anchored to the distal end of the tubular support 21 and the tract 17 of the tubular body 2 is wrapped around the tubular support 21.

At this point the tubular support 21 is inserted into a vessel 4 distally to the zone of the vessel to be substituted or internally lined with the prosthesis 1, or inside a lateral branch of that vessel, in such a way to allow the positioning of the tube 36 in correspondence of a zone of the vessel that can be squeezed around the tube 36 so as to realize the haematic tightness. Then, by acting on the tubular support 21, its sliding relatively to the tube 36 is carried out in order to push the prosthesis along the vessel 4, as shown in detail in figures 12 and 13. The progression of the prosthesis along the blood vessel can be radiologically controlled in order to obtain the correct positioning of the prosthesis with the tract 16 proximal to the segment of the vessel to be substituted or internally lined with the prosthesis.

When the prosthesis is properly positioned, the handles 25 and 26 linked together by the screw 32 are actioned in order to push the cables 27 and 28 along the tubular support 21 in the direction that causes the dilatation of the loops 5 and 6 and then that achieves the adhesion of the prosthesis to the internal walls of the blood vessel (figures 8,9,14). When the degree of radial expansion of the portion 16 of the prosthesis is such that a tightness between the prosthesis and vascular wall is achieved, the tubular support 21 is released from the prosthesis. This release is achieved by unscrewing the screw 32 so that the handle 26 is separated from the handle 25 and then by partially sliding the second cable 28 and then the first cable 27 so as to release these cables from the little cylinder block of manoeuvre 11; at the end the little retaining cable 22 is partially slided causing the release of the support 9 and at the same time the release of the button-hole 39. In this way, the device for the application of the prosthesis is completely released from the prosthesis itself and can be removed from the blood vessel.

It should be noted that anytime during the procedure, by action on the handle 25 and 26 in the reverse way causing reduction of the double loops 5 and 6 to its minimal occupancy, it is possible to correct the prosthesis position inside the vessel or to remove completely the prosthesis.

Whenever the vessel to substituted or internally lined with the prosthesis is surgically exposed it is possible to increase the adhesion of the portion 16 with the vessel walls by tying a ligature around the vessel in correspondence with the expanded portion 16.

When the tract of the vessel is surgically exposed and clamped, the prosthesis can be manually positioned by actioning, instead of the illustrated device, directly on the wires 7 and 8 to achieve the dilatation of the loops 5b and 6b that engages the prosthesis against the vessel internal walls.

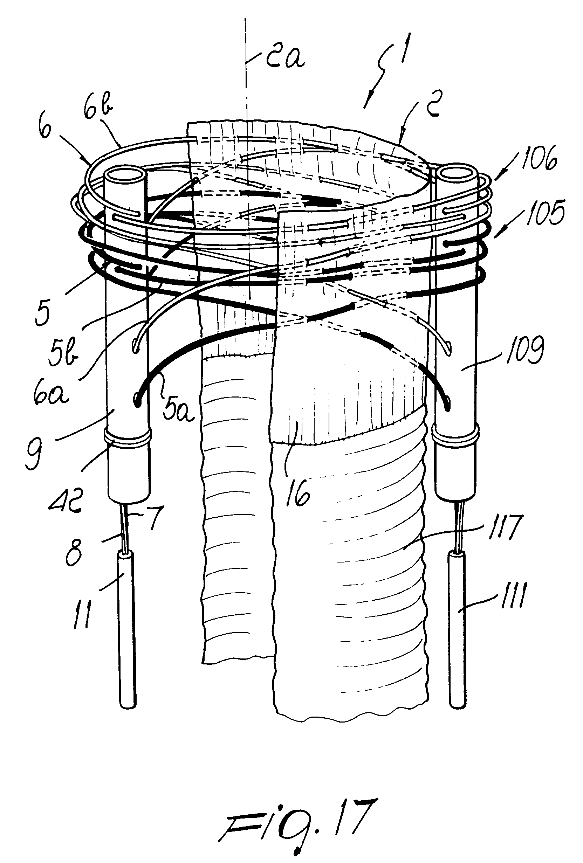

As shown in figure 17, in order to improve the balance of the forces that act on the vessel is advantageous to predispose a second support 109, realized substantially as the support 9, and positioned in a zone diametrically opposite to the support 9 externally to the tubular support 2. The support 109 contains one or more wires 105, 106 with loops disposed substantially as the double loops of the wires 5 and 6 and connected to an other little cylinder block of manoeuvre 11. Also the double loops of wires 105 and 106 are connected to portions of the tubular body 2, as already described in reference to the double loops of wires 5 and 6. The expansion of the double loops 105 and 106 will be obtained by a device as described or by a similar device with two first cables 27 and two second cables 28.

Moreover an annular body 3 can be provided which is similar to that already described, also at the other axial end of the tubular body 2.

In practice it has been observed that the prosthesis according to the invention realizes the prefixed aim, since allows to carry out the substitution or the inner lining of a vascular tract significantly reducing the blood flow interruption phase; moreover when the device for endovascular application is used there is no blood flow interruption phase at all, thus preventing paraplegia due to spinal cord ischemic necrosis, kidney and liver ischemic damages, without requiring by-pass or extracorporeal circulation between the proximal and distal vascular districts and without systemic heparinization, required when extracorporeal circulation is used.

Although the prosthesis according to the invention as well as the device for its application are intended in particular for descending thoracic aorta substitution in case of aneurisms, or dissection, also in rupture phase, and in case of traumatic aortic laceration, it could be advantageously used also for the prosthetic substitution of all tract of artery or vein whose lumen is at least partially patent.

Although the prosthesis according to the invention and the device for its application were prepared for the treatment of aneurisms and traumatic rupture of the thoracic aorta that can be used advantageously also for the prosthetic substitution of any tract of artery or vein whose lumen is at least partially patent.

The prosthesis according to the present invention, as well as the device for its application are susceptible to numerous modifications and variations, all falling within the scope of the inventive concept; furthermore all details may be substituted by technically equivalent elements.

In practising the invention any dimensions or materials may be used, providing that they are compatible with the specific use, according to the contingent requirements and the state of the art.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. Vascular prosthesis for the substitution or internal lining of a segment of blood vessel of medium or large diameter, comprising a tubular body (2) made of biocompatible material including, in proximity of one of its longitudinal ends, at least one body (3) at annular development disposed substantially coaxial to said tubular body (2) and radially expandible for the engagement of a tract of said tubular body (2) containing said body (3) at annular development, against the internal walls of a blood vessel proximal to the segment to be substituted or internally lined, with the prosthesis, said body (3) at annular development being constituted of at least one wire (7, 8) elastically flexible mounted slidingly on a support (9) associated to a lateral portion of said tubular body (2), characterized in that said wire describes, internally to said tract of the tubular body (2), a double loop (5, 6) with a first tract (5a, 6a) coming out from said support(9) along a direction inclined in respect to a plane which is perpendicular to the axis (2a) of said tubular body (2), with a second tract (5b, 6b) at annular development linked to said support in a zone spaced from the zone of coming out of said first tract (5a, 6a), said second tract of the double loops developing in a plane substantially perpendicular to the axis (2a) of said tubular body (2), and with a third tract (5c, 6c) coming back into said support (9) in a zone proximal to the zone of coming out of the said first tract (5a,6a) form the support (9), said wire (7, 8) being sliding relatively to said support (9) in order to vary the amplitude of said double loop (5, 6), tools being provided for contrast of the sliding of said wire relatively to said support in order to maintain the expansion or contraction given to said double loop (5, 6) by the controlled sliding of said wire relatively to said support (9), at the ceasing of the force generating the sliding.

2. Vascular prosthesis, according to claim 1, characterized by the fact that said support (9) is constituted by a substantially cylindric body sited with its axis substantially parallel to the axis (2a) of said tubular body (2), said wires (7, 8) coming out laterally from said support (9) to make said double loops (5, 6).

3. Vascular prosthesis, according to one or more of the above claims, characterized by the fact that the ends of the wires (7, 8) which make said loops come out from an axial end of said support (9) and are jointly associated to a little cylinder block of manoeuvre (11) of said wires, said little cylinder block of manoeuvre (11) being mobile at command relatively to said support (9) for the sliding of said wires (7,8) causing a variation of the amplitude of said loops (5, 6).

4. Vascular prosthesis, according to one or more of the above claims, characterized in that said double loops (5, 6) of the wires (7, 8) and at least the portion of said support (9) involved by said loops (5,6) are connected with a first portion (16) of said tubular body (2), said first portion (16) being constituted by a segment of prosthesis disposed at annular shape and radially expandible, said first portion (16) being associated to the axial end of a second portion (17) substantially cylindric of said tubular body (2) disposed substantially coaxially to said first portion (16).

5. Vascular prosthesis, according to claim 3, characterized in that said little cylinder block of manoeuvre (11) and the portion of said wires extending from said little cylinder block of manoeuvre (11) to said support (9) are disposed externally to said tubular body (2).

6. Vascular prosthesis, according to one or more of the above claims, characterized in that said wires (7, 8) are of harmonic stainless steel.

7. Vascular prosthesis, according to one or more of the previous claims, characterized in that at least a wire includes two wires (7, 8) describing each a double loop (5,6), said first tract (5a, 6a), said second tract (5b, 6b) and said third tract (5c, 6c), of said double loops (5, 6) of said two wires (7, 8) being spaced between them in a direction parallel to the axis (2a) of said tubular body (2).

8. Vascular prosthesis, according to claim 7, characterized by the fact of including tools (10) for positioning of one said double loops (5) in respect to the other (6).

9. Vascular prosthesis, according to claim 8, characterized by the fact that said tools for positioning include segments of wire (10) connecting between them the second tract (5b, 6b) of said double loops (5, 6) in order to keep them in planes substantially parallel between them and perpendicular to the axis (2a) of said tubular body (2).

10. Vascular prosthesis, according to one or more of the preceding claims, characterized by the fact that said tubular body (2) presents an other body at annular development, realized in the same way as said body (3) at annular development, proximal to the other longitudinal end thereof.

11. Vascular prosthesis, according to one or more of the preceding claims characterized in that said body (3) at annular development is constituted by at least two double loops (5, 105) of two wires elastically flexible mounted slidingly each on a support (9, 109) associated to a lateral portion of said tubular body, the two supports (9, 109) being sited in zones of said tubular body diametrically opposite one to the other, each wire of said wires being slidable relatively to the corresponding support (9, 109) in order to vary the amplitude of the related double loop (5, 105), tools being provided for contrast of the sliding of said wires relatively to said supports (9, 109) in order to maintain the expansion or the contraction, given to said double loops (5, 105) by means of the controlled sliding of said wires relatively to said supports (9, 109), at ceasing of the force generating the sliding.

12. Device for the application, without the blood flow interruption, of a vascular prosthesis according to one or more of the above claims, comprising such a vascular prosthesis and a flexible tubular support (21) insertable into the lumen of a blood vessel distal to the tract to be substituted or internally lined with said prosthesis, retaining tools (22) for said support (9), provided at the distal end of said tubular support (21) predisposed to be inserted into the blood vessel, tools for deactivation (23) of said retaining tools (22) for said support (9) being provided in connection with said retaining tools (22), command tools (25,26) provided at the proximal end of said tubular support (21) predisposed to lie externally to said vessel, and connecting tools (27, 28), connected to said command tools (25,26), for connection of said command tools to said at least one wire (7, 8) said command tools including a first handle (25) and a second handle (26) connected to the proximal end of said tubular support (21) and said connection tools including a first cable (27) extending internally to said tubular support (21) for connection to said at least one wire (7,8) and a second cable (28) extending internally to said tubular support (21) to prevent the sliding off of said first cable (27) from said tubular support (21), said first handle (25) being connected to said first cable (27) and said second handle (26) being connected to said second cable (28), said first and second handle (25, 26) being jointly movable relatively to said tubular support (21) for the sliding of said at least one wire (7,8) relatively to said support (9) for a variation of the amplitude of said at least one double loop (5,6) for the radial expansion or contraction of said body (3) at annular development, or individually movable relatively to said tubular support (21) to disconnect said at least one wire form the command tools, thereby leaving the prosthesis internally to the vessel.

13. Device, according to the claim 12, further comprising tools (34,35) for guidance of the prosthesis internally to the blood vessel.

14. Device according claim 13, characterized in that said guiding tools include a cable (34) of guidance connected with one of its ends to said tubular support (21) and suitable to axially cross the prosthesis, the opposite end of said cable (34) of guidance supporting an ogival body (35).

15. Device according to anyone of claims 12-14, characterized in that said tubular support (21) is inserted in internally to a tube (36) partially insertable into said vessel or in lateral branch of said vessel that can be occluded around said tube (36) for the blood tightness during the insertion of the prosthesis, said tubular support being slidable axially, blood-tightly inserted in said tube (36).

16. Device according to one or more of claims 12-15, characterized in that said retaining tools include a retaining cable (22) slidable internally to said tubular support (21), said retaining cable (22) connecting with its distal end said tubular support (21) to said support (9), said retaining cable (22) coming out with its proximal end from said tubular support (21) externally to the vessel and being slidable at command along said tubular support (21) to release said support (9) from the distal end of said tubular support (21).

17. Device according to one or more of claims 12-16, characterized by the fact of including tools (39, 40) for the fixation of the prosthesis to the lateral surface of said tubular support (21).

18. Device according to one or more of claims 12-17, characterized in that said fixation tools include a fixing button-hole (39) connected to said prosthesis and insertable into a fissure (40) defined in the lateral surface of said tubular support (21), said button-hole (39) being crossable by a retaining cable (22) for retention of said prosthesis in proximity of said tubular body.

19. Device according to one or more claims 12-18, characterized by the fact of including tools (48) for tightness interposed between said cables and the internal surface of said tubular support.

## Patentansprüche

1. Gefäßprothese zum Ersetzen oder inneren Auskleiden eines Blutgefäßsegmentes von mittlerem oder großem Durchmesser, aufweisend einen röhrenförmigen Körper (2) aus biokompatiblem Material einschließlich, in der Nähe eines seiner longitudinalen Enden, mindestens eines Körpers (3) zur ringförmigen Erweiterung, der im wesentlichen koaxial zum röhrenförmigen Körper (2) angeordnet ist und zum Angriff eines Stranges des röhrenförmigen Körpers (2), der den Körper (3) zur ringförmigen Erweiterung enthält, gegen die Innenwände eines Blutgefäßes radial ausdehnbar ist, das benachbart dem durch die Prothese zu ersetzenden oder innerlich auszukleidenden Segment ist, wobei der Körper (3) zur ringförmigen Erweiterung von mindestens einem elastisch flexiblen Draht (7, 8) gebildet wird, der verschiebbar auf einem Träger (9) angebracht ist, welcher mit einem seitlichen Abschnitt des röhrenförmigen Körpers (2) in Verbindung steht, dadurch gekennzeichnet, daß der Draht innerhalb des Stranges des röhrenförmigen Körpers (2) eine Doppelschleife (5, 6) beschreibt, wobei ein erster Strang (5a, 6a) entlang einer Richtung, die in bezug auf eine senkrecht zur Achse (2a) des röhrenförmigen Körpers (2) liegende Ebene geneigt ist, aus dem Träger (9) herauskommt, wobei ein zweiter Strang (5b, 6b) zur ringförmigen Erweiterung in einer Zone mit dem Träger verbunden ist, welche von der Zone des Herauskommens des ersten Stranges (5a, 6a) beabstandet ist, wobei sich der zweite Strang der Doppelschleife in einer Ebene erweitert, die im wesentlichen senkrecht zur Achse (2a) des röhrenförmigen Körpers (2) ist, und wobei ein dritter Strang (5c, 6c) in den Träger (9) an einer Zone, die der Zone des Herauskommens des ersten Stranges (5a, 6a) aus dem Träger (9) benachbart ist, in den Träger (9) zurückkommt, wobei der Draht (7, 8) relativ zum Träger (9) verschiebbar ist, um die Amplitude der Doppelschleife (5, 6) zu variieren, wobei Einrichtungen vorgesehen sind, um dem Verschieben des Drahtes relativ zum Träger entgegenzuwirken, um die Ausdehnung oder die Kontraktion, die der Doppelschleife (5, 6) durch das kontrollierte Verschieben des Drahtes relativ zum Träger (9) verliehen wird, aufrechtzuerhalten, wenn die die Verschiebung erzeugende Kraft zu wirken aufhört.

2. Gefäßprothese nach Anspruch 1, dadurch gekennzeichnet, daß der Träger (9) von einem im wesentlichen zylindrischen Körper gebildet wird, der so gelegen ist, daß seine Achse im wesentlichen parallel zur Achse (2a) des röhrenförmigen Körpers (2) ist, wobei die Drähte (7, 8) seitlich aus dem Träger (9) herauskommen, um die Doppelschleifen (5, 6) zu bilden.

3. Gefäßprothese nach mindestens einem der vorstehenden Ansprüche, dadurch gekennzeichnet, daß die Enden der Drähte (7, 8), die die Schleifen bilden, aus einem axialen Ende des Trägers (9) herauskommen und gemeinsam mit einem kleinen zylindrischen Betätigungsblock (11) der Drähte in Verbindung stehen, wobei der kleine zylindrische Betätigungsblock (11) auf eine Steuerung hin relativ zum Träger (9) für das Verschieben der Drähte (7, 8) beweglich ist, was eine Variation der Amplitude der Schleifen (5, 6) bewirkt.

4. Gefäßprothese nach mindestens einem der vorstehenden Ansprüche, dadurch gekennzeichnet, daß die Doppelschleifen (5, 6) der Drähte (7, 8) und mindestens der Abschnitt des Trägers (9), der durch die Schleifen (5, 6) eingeschlossen ist, mit einem ersten Abschnitt (16) des röhrenförmigen Körpers (2) verbunden sind, wobei der erste Abschnitt (16) von einem Prothesensegment gebildet wird, das ringförmig und radial ausdehnbar angeordnet ist, wobei der erste Abschnitt (16) mit dem axialen Ende eines im wesentlichen zylindrischen zweiten Abschnittes (17) des röhrenförmigen Körpers (2), der im wesentlichen koaxial zum ersten Abschnitt (16) angeordnet ist, in Verbindung steht.

5. Gefäßprothese nach Anspruch 3, dadurch gekennzeichnet, daß der kleine zylindrische Betätigungsblock (11) und der Abschnitt der Drähte, der sich vom kleinen zylindrischen Betätigungsblock (11) zum Träger (9) erstreckt, außerhalb des röhrenförmigen Körpers (2) angeordnet sind.

6. Gefäßprothese nach mindestens einem der vorstehenden Ansprüche, dadurch gekennzeichnet, daß die Drähte (7, 8) aus harmonischem rostfreien Stahl sind.

7. Gefäßprothese nach mindestens einem der vorstehenden Ansprüche, dadurch gekennzeichnet, daß mindestens ein Draht zwei Drähte (7, 8) umfaßt, von denen jeder eine Doppelschleife (5, 6) beschreibt, wobei der erste Strang (5a, 6a), der zweite Strang (5b, 6b) und der dritte Strang (5c, 6c) der Doppelschleifen (5, 6) der beiden Drähte (7, 8) zwischen ihnen in einer Richtung, die parallel zur Achse (2a) des röhrenförmigen Körpers (2) liegt, beabstandet sind.

8. Gefäßprothese nach Anspruch 7, dadurch gekennzeichnet, daß sie Einrichtungen (10) zum Positionieren einer der Doppelschleifen (5) in bezug auf die andere (6) umfaßt.

9. Gefäßprothese nach Anspruch 8, dadurch gekennzeichnet, daß die Einrichtungen zum Positionieren Drahtsegmente (10) umfassen, die zwischen sich den zweiten Strang (5b, 6b) der Doppelschleifen (5, 6) verbinden, um sie in Ebenen zu halten, welche im wesentlichen parallel zwischen ihnen und senkrecht zur Achse (2a) des röhrenförmigen Körpers (2) liegen.

10. Gefäßprothese nach mindestens einem der vorstehenden Ansprüche, dadurch gekennzeichnet, daß der röhrenförmige Körper (2) in der Nähe seines anderen longitudinalen Endes einen weiteren Körper zur ringförmigen Erweiterung aufweist, der in der gleichen Weise wie der Körper (3) zur ringförmigen Erweiterung ausgeführt ist.

11. Gefäßprothese nach mindestens einem der vorstehenden Ansprüche, dadurch gekennzeichnet, daß der Körper (3) zur ringförmigen Erweiterung von mindestens zwei Doppelschleifen (5, 105) aus zwei elastisch flexiblen Drähten gebildet wird, von denen jeder verschiebbar auf einem Träger (9, 109) angebracht ist, der mit einem seitlichen Abschnitt des röhrenförmigen Körpers in Verbindung steht, wobei die beiden Träger (9, 109) in Zonen des röhrenförmigen Körpers gelegen sind, welche einander diametral gegenüberliegen, wobei jeder Draht der Drähte relativ zum entsprechenden Träger (9, 109) verschiebbar ist, um die Amplitude der zugehörigen Doppelschleife (5, 105) zu variieren, wobei Einrichtungen vorgesehen sind, um dem Verschieben der Drähte relativ zu den Trägern (9, 109) entgegenzuwirken, um die Ausdehnung oder die Kontraktion, die den Doppelschleifen (5, 105) durch das kontrollierte Verschieben der Drähte relativ zu den Trägern (9, 109) verliehen wird, aufrechtzuerhalten, wenn die die Verschiebung erzeugende Kraft zu wirken aufhört.

12. Vorrichtung zum Anbringen einer Gefäßprothese gemäß einem oder mehreren der vorstehenden Ansprüche ohne die Unterbrechung des Blutflusses, aufweisend eine derartige Gefäßprothese und einen flexiblen röhrenförmigen Träger (21), der in das Lumen eines Blutgefäßes, welches zu dem mit der Prothese zu ersetzenden oder innerlich auszukleidenden Strang distal ist, einschiebbar ist, Rückhalteeinrichtungen (22) für den Träger (9), die am distalen Ende des röhrenförmigen Trägers (21) vorgesehen sind, welcher dafür eingerichtet ist, in das Blutgefäß eingeführt zu werden, wobei Deaktivierungseinrichtungen (23) der Rückhalteeinrichtungen (22) für den Träger (9) zusammen mit den Rückhalteeinrichtungen (22) vorgesehen sind, Steuereinrichtungen (25, 26), die am proximalen Ende des röhrenförmigen Trägers (21) vorgesehen sind, welcher dafür eingerichtet ist, außerhalb des Gefäßes zu liegen, und mit den Steuereinrichtungen (25, 26) verbundene Verbindungseinrichtungen (27, 28) zur Verbindung der Steuereinrichtungen mit mindestens einem Draht (7, 8), wobei die Steuereinrichtungen einen ersten Handgriff (25) und einen zweiten Handgriff (26) umfassen, die mit dem proximalen Ende des röhrenförmigen Trägers (21) verbunden sind, und wobei die Verbindungseinrichtungen ein sich innerhalb des röhrenförmigen Trägers (21) erstreckendes erstes Kabel (27) zur Verbindung mit dem mindestens einen Draht (7, 8) und ein sich innerhalb des röhrenförmigen Trägers (21) erstreckendes Zweites Kabel (28) zum Vermeiden des Herausgleitens des ersten Kabels (27) aus dem röhrenförmigen Träger (21) umfassen, wobei der erste Handgriff (25) mit dem ersten Kabel (27) verbunden ist und der zweite Handgriff (26) mit dem zweiten Kabel (28) verbunden ist, wobei der erste und zweite Handgriff (25, 26) gemeinsam relativ zum röhrenförmigen Träger (21) für das Verschieben des mindestens einen Drahtes (7, 8) relativ zum Träger (9) für eine Variation der Amplitude der mindestens einen Doppelschleife (5, 6) für das radiale Ausdehnen oder Zusammenziehen des Körpers (3) zur ringförmigen Erweiterung beweglich sind oder individuell relativ zum röhrenförmigen Träger (21) beweglich sind, um den mindestens einen Draht von den Steuereinrichtungen zu lösen, wodurch die Prothese innerhalb des Gefäßes gelassen wird.

13. Vorrichtung nach Anspruch 12, welche Werkzeuge (34, 35) für die Führung der Prothese innerhalb des Blutgefäßes aufweist.

14. Vorrichtung nach Anspruch 13, dadurch gekennzeichnet, daß die Führungswerkzeuge ein Führungskabel (34) umfassen, das mit einem seiner Enden mit dem röhrenförmigen Träger (21) verbunden ist und das dafür geeignet ist, die Prothese axial zu kreuzen, wobei das gegenüberliegende Ende des Führungskabels (34) einen spitzkegelförmigen Körper (35) trägt.

15. Vorrichtung nach einem der Ansprüche 12 bis 14, dadurch gekennzeichnet, daß der röhrenförmige Träger (21) in eine Röhre (36) eingeführt ist, die teilweise in das Gefäß oder in einen seitlichen Zweig des Gefäßes, das um die Röhre (36) herum für die Blutdichtigkeit während des Einschubs der Prothese verschlossen werden kann, einschiebbar ist, wobei der röhrenförmige Träger axial verschiebbar und blutdicht in die Röhre (36) eingeschoben ist.

16. Vorrichtung nach mindestens einem der Ansprüche 12 bis 15, dadurch gekennzeichnet, daß die Rückhalteeinrichtungen ein Rückhaltekabel (22) umfassen, das innerhalb des röhrenförmigen Trägers (21) verschiebbar ist, wobei das Rückhaltekabel (22) mit seinem distalen Ende den röhrenförmigen Träger (21) mit dem Träger (9) verbindet, wobei das Rückhalrekabel (22) mit seinem proximalen Ende aus dem röhrenförmigen Träger (21) außerhalb des Gefäßes herauskommt und auf eine Steuerung hin entlang dem röhrenförmigen Träger (21) verschiebbar ist, um den Träger (9) vom distalen Ende des röhrenförmigen Trägers (21) zu lösen.

17. Vorrichtung nach mindestens einem der Ansprüche 12 bis 16, dadurch gekennzeichnet, daß sie Einrichtungen (39, 40) für die Befestigung der Prothese an der seitlichen Oberfläche des röhrenförmigen Trägers (21) umfaßt.

18. Vorrichtung nach mindestens einem der Ansprüche 12 bis 17, dadurch gekennzeichnet, daß die Befestigungseinrichtungen ein Befestigungsknopfloch (39) umfassen, das mit der Prothese verbunden ist und in eine Spalte (40), die in der seitlichen Oberfläche des röhrenförmigen Trägers (21) begrenzt ist, einschiebbar ist, wobei das Knopfloch (39) von einem Rückhaltekabel (22) zum Halten der Prothese in der Nähe des röhrenförmigen Körpers durchquerbar ist.

19. Vorrichtung nach mindestens einem der Ansprüche 12 bis 18, dadurch gekennzeichnet, daß sie Einrichtungen (48) für einen festen Sitz umfaßt, die zwischen die Kabel und die innere Oberfläche des röhrenförmigen Trägers gesetzt sind.

## Revendications

1. Prothèse vasculaire pour la substitution ou le revêtement interne d'un segment de vaisseau sanguin de grand ou moyen diamètre, comprenant un corps tubulaire (2) réalisé en une matière biocompatible incluant, à proximité de l'une de ses extrémités longitudinales, au moins un corps (3) à développement annulaire disposé sensiblement coaxialement audit corps tubulaire (2) et expansible radialement pour l'engagement d'une partie dudit corps tubulaire (2) contenant ledit corps (3) à développement annulaire, contre la paroi interne d'un vaisseau sanguin proche du segment à remplacer ou à revêtir intérieurement, avec la prothèse, ledit corps (3) à développement annulaire étant constitué d'au moins un fil métallique (7,8) élastiquement souple, monté de façon coulissante sur un support (9) associé à une portion latérale dudit corps tubulaire (2) ,
caractérisée en ce que ledit fil métallique décrit, intérieurement à ladite partie du corps tubulaire (2), une double boucle (5,6) ayant une première partie (5a,6a) sortant dudit support (9) le long d'une direction inclinée par rapport à un plan qui est perpendiculaire à l'axe (2a) dudit corps tubulaire (2), une deuxième partie (5b,6b) à développement annulaire liée audit support dans une zone espacée de la zone de sortie de ladite première partie (5a,6a), ladite deuxième partie de la double boucle se développant dans un plan sensiblement perpendiculaire à l'axe (2a) dudit corps tubulaire (2), et une troisième partie (5c,6c) revenant dans ledit support (9) dans une zone proche de la zone de sortie de ladite première partie (5a,6a) du support (9), ledit fil métallique (7,8) étant coulissant par rapport audit support (9) de façon à varier l'amplitude de ladite double boucle (5,6), des outils étant prévus pour s'opposer au coulissement dudit fil métallique par rapport audit support de façon à maintenir l'expansion ou la rétraction donnée à ladite double boucle (5,6) par le coulissement contrôlé dudit fil métallique par rapport audit support (9), lorsque cesse la force engendrant le coulissement.

2. Prothèse vasculaire selon la revendication 1,
caractérisée par le fait que ledit support (9) est constitué par un corps sensiblement cylindrique placé avec son axe sensiblement parallèle à l'axe (2a) dudit corps tubulaire (2), lesdits fils métalliques (7,8) sortant latéralement dudit support (9) pour réaliser lesdites doubles boucles (5,6).

3. Prothèse vasculaire selon l'une ou plusieurs des revendications précédentes,
caractérisée par le fait que les extrémités des fils métalliques (7,8) qui forment lesdites boucles, sortent d'une extrémité axiale dudit support (9) et sont associées de façon jointive à un petit bloc cylindrique de manoeuvre (11) desdits fils, ledit petit bloc cylindrique de manoeuvre (11) étant mobile à la commande par rapport audit support (9) pour le coulissement desdits fils (7,8) provoquant une variation de l'amplitude desdites boucles (5,6).

4. Prothèse vasculaire selon l'une ou plusieurs des revendications précédentes,
caractérisée en ce que lesdites doubles boucles (5,6) des fils métalliques (7,8) et au moins la portion dudit support (9) impliquée par lesdites boucles (5,6) sont reliées à une première portion (16) dudit corps tubulaire (2), ladite première portion (16) étant constituée par un segment de prothèse agencé sous forme annulaire et radialement expansible, ladite première portion (16) étant associée à l'extrémité axiale d'une seconde porton (17) sensiblement cylindrique dudit corps tubulaire (2) disposé sensiblement coaxialement à ladite première portion (16).

5. Prothèse vasculaire selon la revendication 3,
caractérisée en ce que ledit petit bloc cylindrique de manoeuvre (11) et la portion desdits fils métalliques s'étendant à partir dudit petit bloc cylindrique de manoeuvre (11) vers ledit support (9), sont disposés extérieurement audit corps tubulaire (2).

6. Prothèse vasculaire selon l'une ou plusieurs des revendications précédentes,
caractérisée en ce que lesdits fils métalliques (7,8) sont en acier inoxydable harmonique.

7. Prothèse vasculaire selon l'une ou plusieurs des revendications précédentes,
caractérisée en ce qu'au moins un fil métallique inclut deux fils (7,8) décrivant chacun une double boucle (5,6), ladite première partie (5a,6a), ladite deuxième partie (5b,6b) et ladite troisième partie (5c,6c) desdites doubles boucles (5,6) desdits deux fils (7,8) étant espacées entre elles dans une direction parallèle à l'axe (2a) dudit corps tubulaire (2).

8. Prothèse vasculaire selon la revendication 7,
caractérisée par le fait d'inclure des outils (10) pour positionner l'une desdites doubles boucles (5) par rapport à l'autre boucle (6).

9. Prothèse vasculaire selon la revendication 8,
caractérisée par le fait que lesdits outils de positionnement comprennent des segments de fil métallique (10) reliant entre eux la deuxième partie (5b,6b) desdites doubles boucles (5,6) de façon à les maintenir dans des plans sensiblement parallèles entre eux et perpendiculaires à l'axe (2a) dudit corps tubulaire (2).

10. Prothèse vasculaire selon l'une ou plusieurs des revendications précédentes,
caractérisée par le fait que ledit corps tubulaire (2) présente un autre corps à développement annulaire, réalisé de la même façon que ledit corps (3) à développement annulaire, proche de l'autre extrémité longitudinale de celui-ci.

11. Prothèse vasculaire selon l'une ou plusieurs des revendications précédentes,
caractérisée en ce que ledit corps (3) à développement annulaire est constitué par au moins deux doubles boucles (5,105) de deux fils métalliques élastiquement souples montés chacun, de façon coulissante, sur un support (9,109) associe à une portion latérale dudit corps tubulaire, les deux supports (9,109) étant situés dans des zones dudit corps tubulaire diamétralement en opposition l'une de l'autre, chaque fil desdits fils métalliques pouvant coulisser par rapport au support correspondant (9,109) de façon à faire varier l'amplitude de la double boucle concernée (5,105), des outils étant prévus pour s'opposer au coulissement desdits fils métalliques par rapport auxdits supports (9,109) de façon à maintenir l'expansion ou la rétraction, donnée par lesdites doubles boucles (5,105) par l'intermédiaire du coulissement contrôlé desdits fils métalliques par rapport auxdits supports (9,109), lorsque cesse la force engendrant le coulissement.

12. Dispositif pour l'application, sans l'interruption de la circulation sanguine, d'une prothèse vasculaire selon l'une ou plusieurs des revendications précédentes, comprenant une telle prothèse vasculaire et un support tubulaire souple (21) insérable dans le passage d'un vaisseau sanguin à distance de la partie à substituer ou à revêtir intérieurement avec ladite prothèse, des outils de retenue (22) pour ledit support (9), prévus à l'extrémité distale dudit support tubulaire (21) prédisposé pour être inséré dans le vaisseau sangun, des outils (23) pour désactiver lesdits outils de retenue (22) pour ledit support (9) étant prévus en liaison avec lesdits outils de retenue (22), des outils de commande (25,26) prévus à l'extrémité proximale dudit support tubulaire (21) prédisposés pour s'étendre extérieurement audit vaisseau, et des outils de connexion (27,28) reliés auxdits outils de commande (25,26) pour la liaison desdits outils de commande à au moins un fil métallique (7,8), lesdits outils de commande incluant une première poignée (25) et une seconde poignée (26) reliées à l'extrémité proximale dudit support tubulaire (21) et lesdits outils de connexion incluant un premier câble (27) s'étendant intérieurement audit support tubulaire (21) pour relier ledit fil métallique (7,8), et un second câble (28) s'étendant intérieurement audit support tubulaire (21) pour empêcher la sortie dudit premier câble (27) dudit support tubutaire (21), ladite première poignée (25) étant reliée audit premier câble (27) et ladite seconde poignée (26) étant reliée audit second câble (28), lesdites première et seconde poignées (25,26) étant déplaçables de façon jointive par rapport audit support tubulaire (21) pour le coulissement dudit fil métallique (7,8) par rapport audit support (9) pour une variation de l'amplitude d'au moins une double boucle (5,6) pour l'expansion ou la rétraction radiale dudit corps (3) à développement annulaire, ou déplacables individuellement par rapport audit support tubulaire (21) pour déconnecter ledit fil métallique des outils de commande, en laissant la prothèse à l'intérieur du vaisseau.

13. Dispositif selon la revendication 12, comprenant de plus des outils (34,35) pour guider la prothèse à l'intérieur du vaisseau sanguin.

14. Dispositif selon la revendication 13,
caractérisé en ce que lesdits outils de guidage comprennent un câble de guidage (34) relié, à l'une de ses extrémités, audit support tubulaire (21) et adapté pour traverser axialement la prothèse, l'extrémité opposée dudit câble de guidage (34) supportant un corps ogival (35).

15. Dispositif selon l'une quelconque des revendications 12-14,
caractérisé en ce que ledit support tubulaire (21) est inséré à l'intérieur d'un tube (36) partiellement insérable dans ledit vaisseau ou dans une branche latérale dudit vaisseau qui peut être obturé autour dudit tube (36) pour l'étanchéité sanguine pendant l'insertion de la prothèse, ledit support tubulaire étant coulissable axialement, inséré de façon étanche au sang dans ledit tube (36).

16. Dispositif selon l'une ou plusieurs des revendications 12-15,
caractérise en ce que lesdits outils de retenue comportent un câble de retenue (22) coulissable à l'intérieur dudit support tubulaire (21), ledit câble de retenue (22) reliant, par son extrémité distale, ledit support tubulaire (21) audit support (9), ledit câble de retenue (22) sortant avec son extrémité proximale dudit support tubulaire (21) extérieurement au vaisseau et étant coulissable, par commande, le long dudit support tubulaire (21) pour libérer ledit support (9) de l'extrémité distale dudit support tubulaire (21).

17. Dispositif selon l'une ou plusieurs des revendications 12-16,
caractérisé par le fait d'inclure des outils (39, 40) pour la fixation de la prothèse à la surface latérale dudit support tubulaire (21).

18. Dispositif selon l'une ou plusieurs des revendications 12-17,
caractérisé en ce que lesdits outils de fixation comportent une boutonnière de fixation (39) reliée à ladite prothèse et insérable dans une fissure (40) définie dans la surface latérale dudit support tubulaire (21), ladite boutonnière (39) pouvant être traversée par un câble de retenue (22) pour maintenir ladite prothèse à proximité dudit corps tubulaire.

19. Dispositif selon l'une ou plusieurs des revendications 12-18,
caractérisé par le fait d'inclure des outils (48) pour l'étanchéité, disposés entre lesdits câbles et la surface interne dudit support tubulaire.
